# EUROPEAN PATENT APPLICATION

(11) **EP 3 854 427 A1**
(43) Date of publication of application: **28.07.2021**
(21) Application number: 21151601.8
(22) Date of filing: 18.01.2018
(51) Int. Cl.: A61L 29/08

(54) **METHODS OF STERILIZING A HYDROPHILICALLY COATED MEDICAL DEVICE**

(30) Priority: 20.01.2017 US 201762448708 P
(62) Divisional of application: 18703146.3
(71) Applicant: Hollister Incorporated, Libertyville IL 60048 (US)
(72) Inventor: MONTES DE OCA, Horacio, Libertyville, IL Illinois 60048 (US); HENRY, Jerome A., Libertyville, IL Illinois 60048 (US); ROSTAMI, Shamsedin, Libertyville, IL Illinois 60048 (US)
(74) Representative: FRKelly

(57) **Abstract**

Method for sterilizing a hydrophilically coated medical device.

## Description

### Related Application

The present application claims the benefit and priority of U.S. Provisional Patent Application No. 62/448,708, filed January 20, 2017, which is hereby incorporated herein by reference.

### TECHNICAL FIELD

The present disclosure generally relates to methods of radiation sterilizing a hydrophilically coated medical device in a hydration environment, and more particularly, to methods for radiation sterilization of hydrophilic coatings in a hydration environment that include applying a protective coating over the hydrophilic coating prior to exposing the hydrophilic coating to sterilizing radiation.

### BACKGROUND

It is known to coat medical devices, such as urinary catheters, with a hydrophilic coating. When the hydrophilic coating is wetted or hydrated with a wetting fluid, such as water, it becomes extremely lubricous which eases introduction of the device into the body and aids in reducing pain and discomfort associated with such introduction.

In some applications, the hydrophilically coated medical device is provided in a "dry" state wherein the user is required to wet the hydrophilic coating with a wetting fluid immediately prior to insertion into the body. In other applications, it is desirable to provide a hydrophilically coated medical device that is in a ready-to-use condition right out of the package. In the field of urinary catheters, a hydrophilically coated catheter may be provided in a catheter package that has a hydration environment that hydrates/wets the catheter. In one type of catheter assembly, the hydrophilically coated catheter is stored in the package in contact with liquid water so that the hydrophilic coating is wetted within the package and the catheter is ready for use right out of the package. In other catheter package assemblies, the hydrophilically coated catheter may be provided in a catheter package that has a vapor hydration atmosphere wherein the hydrophilic coating is wetted by water vapor so that the catheter is ready to use right out of package for the end user.

For various reasons, including but not limited to efficiency, effectiveness and cost, it is desirable to radiation sterilize packaged medical device assemblies. In some instances, the hydrophilically coated medical device and liquid water are placed in the package and the package is sealed. The liquid water wets/hydrates the hydrophilic coating by being in direct contact with the hydrophilic coating or the water may provide a water vapor which wets/hydrates the hydrophilic coating. After the package is sealed, the package having the hydrophilically coated medical device and water (liquid and/or vapor) therein is exposed to radiation, such as gamma or E-Beam radiation, to sterilize the medical device. It has been found, however, that sterilization of hydrophilic coatings in the hydrated state or while in contact with a wetting fluid can result in degradation of the coating or excessive crosslinking that can lead to an increase of coefficient of friction (decrease in lubricity) of the coating and/or cause instability of the coating which may result in the coating undesirably detaching from the medical device prior to or during use.

Therefore, there remains a need for methods of sterilizing medical devices having hydrophilic coatings.

### SUMMARY

In one aspect, a method of sterilizing a medical device that includes a hydrophilic surface, the method includes applying a protective coating over a hydrophilic surface of the medical device, placing the medical device in a hydration environment, and exposing the medical device to sterilizing radiation.

In another aspect, a method of making a ready to use hydrophilically coated medical device assembly that includes applying a protective coating over a hydrophilic coating of the medical device, placing the medical device into a package along with liquid water, sealing the package, and exposing the medical device assembly to sterilizing radiation.

In another aspect, a hydrophilically coated medical assembly that includes a package containing a medical device having a hydrophilic coating thereon and a protective coating over the hydrophilic coating. The package also contains an amount of water.

### DESCRIPTION

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

The present disclosure relates to methods for sterilizing medical devices having a hydrophilic surface, such as a medical device having a hydrophilic coating on a surface of the medical device. Such methods may include applying a protective coating over the hydrophilic surface of the medical device, placing the medical device into a hydration environment, and then exposing the medical device to a sufficient amount of sterilization radiation, such as gamma or E-Beam radiation. The hydration environment may be an environment wherein the medical device is in direct contact with liquid water and/or wherein the medical device is exposed to water vapor.

The protective coating may temporarily prevent the hydrophilic surface from being hydrated for a desired period of time or temporarily limit the level of hydration for a desired period of time. In other words, the protective coating may temporarily prevent or limit liquid water and/or water vapor from coming into contact with and being absorbed into the hydrophilic material. In one embodiment, the protective coating limits/prevents hydration of a hydrophilic surface for a time period sufficient to allow the medical device to be radiation sterilized prior to hydration of the hydrophilic material. It is understood, without being held to any particular theory, that the protective coating coats the surface of the hydrophilic material and is absorbed into the interstices of the hydrophilic material's polymer matrix, thereby repelling the absorption of water into the hydrophilic coating, i.e., preventing or limiting the amount of liquid or vapor water from entering the hydrophilic polymer matrix. This causes a delay in the hydration of the hydrophilic material for a desired period of time, during which the medical device is exposed to sterilizing radiation. Eventually, liquid water or water vapor permeates through/past the protective coating and/or displaces at least some of the protective coating, thereby hydrating the hydrophilic material so that it is in a ready-to-use condition for the end user.

In one embodiment, the protective coating is a liquid that has a viscosity between about 30cP to about 60cP at 25°C. The protective coating may be applied in any suitable manner, such as by dip coating, spraying or painting. The amount or level of coating can vary depending on the viscosity of the protective coating, the dwell time, dip time or amount of protective coating applied by spraying or painting. The protective coating may include, for example, one or more polyols. Such polyols may include, but are not limited to, 3-carbon sugar alcohols (Glycerol); 4-carbon sugar alcohols (Erythritol, Threitol); 5-carbon sugar alcohols (Arabitol, Xylitol, Adonitol), 6-carbon sugar alcohols (Mannitol, Sorbitol, Galactitol, Fucitol, Iditol, Inositol), 7-carbon sugar alcohols (Volemitol),12-carbon sugar alcohols (Isomalt, Maltitol, Lactitol), 18-carbon sugar alcohols (Maltotriitol), and 24-carbon sugar alcohols (Maltotetraitol), propylene glycol, tetraethylene glycol.

When the hydrophilic surface of the medical device is a hydrophilic coating, the hydrophilic coating may include a hydrophilic polymer, among other components, including but not limited to antioxidants, polyelectrolytes, plasticizers and the like. The hydrophilic polymer may be, for example, polyvinylpyrrolidone (PVP), polyethylene oxide, methyl cellulose, ethyl cellulose, polyethylene glycol, hydroxyl methylcellulose, hydroxypropyl cellulose, hydroxypropyl methylcellulose, carboxymethyl cellulose, polyvinyl alcohol, or mixtures thereof. Furthermore, when formed on the medical device, the hydrophilic coating may be a crosslinked hydrophilic coating.

In one method of forming a sterilized ready-to-use hydrophilically coated medical device assembly, a protective coating is applied over the hydrophilic surface, such as a hydrophilic coating, of the medical device by, for example, dipping the medical device into the protective coating. In one embodiment, the hydrophilic coating may be a crosslinked hydrophilic coating that was applied to the medical device in any suitable manner. In one embodiment, a hydrophilically coated urinary catheter may be dip coated with a polyol, such as glycerol. The hydrophilically coated medical device is then placed within a hydration environment. For example, the medical device may be placed into a package along with liquid water and the package may then be sealed. In one embodiment, after the catheter is coated with a polyol, the catheter may be positioned in a sleeve that contains water and the sleeve may be the package or the sleeve may be placed in an outer package. The sleeve may be, for example, a no-touch sleeve wherein the user uses the sleeve to insert the catheter. The liquid water may be in direct contact with the medical device and/or the liquid water may produce a water vapor that is in contact with the medical device. The package may be made from a material that is liquid and gas impermeable. Furthermore, in one embodiment wherein the liquid water produces a water vapor for hydrating the hydrophilic coating, the liquid water is kept separated from the medical device. For instance, the liquid water may be retained by a liquid sequestering element, such as an absorbent member that holds the water or a compartment that is at least partially made from a liquid impermeable, gas permeable material.

After the medical device is placed in the hydration environment, the medical device is exposed to sterilization radiation. For example, after the medical device is placed in the package along with liquid water and the package is sealed, the package is exposed to sterilizing radiation, such as E-beam or gamma radiation. The distribution of the package may then be delayed for a time sufficient for the hydration environment to hydrate the hydrophilic coating, i.e., sufficient amount of time to allow the liquid and/or vapor water to permeate through the protective coating to hydrate the device. In one embodiment, the packages may be stored for a period of time after radiation and then distributed.

As can be seen from the above description, the present disclosure has several different aspects, which are not limited to the specific aspects and which do not necessarily need to be used together. Variations of these concepts or structures may be embodied in other structures without departing from the present invention as set forth in the appended claims.

Additional features of the invention are set out below.
1. A method of sterilizing a medical device having a hydrophilic surface, comprising:
   applying a protective coating over a hydrophilic surface of a medical device;
   placing the medical device in a hydration environment; and
   exposing the medical device to sterilizing radiation.
2. The method of feature 1 wherein the hydrophilic surface comprises a hydrophilic coating disposed on the medical device.
3. The method of feature 2 wherein the hydrophilic coating comprises a crosslinked hydrophilic polymer.
4. The method of any one of the preceding features wherein the protective coating comprises a polyol.
5. The method of feature 4 wherein the polyol comprises a carbon chain including between 3 and 24 carbon atoms.
6. The method of any one of the preceding features wherein the protective coating comprises one or more of glycerol, propylene glycol and tetraethylene glycol.
7. The method of any one of the preceding features wherein the hydrophilic surface comprises polyvinylpyrrolidone.
8. The method of any one of the preceding features wherein the sterilizing radiation comprises E-beam or gamma radiation.
9. The method of any one of the preceding features wherein the placing the medial device within a hydration environment comprises placing the catheter in a package containing a hydration fluid.
10. The method of feature 8 wherein the package is sealed prior to exposing the medical device to sterilizing radiation.
11. The method of any one of the preceding features wherein the medical device is a urinary catheter.
12. A method of making a ready to use hydrophilically coated medical device assembly, comprising:
   applying a protective coating over a hydrophilic coating of a medical device;
   placing the medical device into a package along with liquid water;
   sealing the package; and
   exposing the medical device assembly to sterilizing radiation.
13. The method of feature 12 further including delaying the distribution of the medical device assembly for a time period sufficient to allow hydration of the hydrophilic coating to occur.
14. The method of any one of features 12 and 13 wherein the liquid water is in direct liquid contact with the medical device.
15. The method of any one of features 12 and 13 wherein the liquid water produces a water vapor that hydrates the medical device.
16. The method of feature 15 wherein the liquid water is separated from the medical device.
17. The method of any one of features 12-16 wherein the protective coating comprises a polyol.
18. The method of any one of the features 12-17 wherein the polyol comprises one or more of glycerol, propylene glycol and tetraethylene glycol.
19. The method of any one claims wherein 12-18 the hydrophilic coating comprises polyvinylpyrrolidone.
20. The method of any one of features 12-19 wherein the medical device is a urinary catheter.
21. A hydrophilically coated medical assembly, comprising:
   a package containing:
   a medical device having a hydrophilic coating thereon and a protective coating over the hydrophilic coating; and
   an amount of water.
22. The assembly of feature 21 wherein the liquid water is in direct liquid contact with the medical device.
23. The assembly of feature 21 wherein the liquid water produces a water vapor that hydrates the medical device.
24. The assembly of feature 22 wherein the liquid water is separated from the medical device.
25. The assembly of any one of features 21-24 wherein the protective coating comprises a polyol.
26. The assembly of any one of the features 21-25 wherein the polyol comprises one or more of glycerol, propylene glycol and tetraethylene glycol.
27. The assembly of any one features wherein 21-26 the hydrophilic coating comprises polyvinylpyrrolidone.
28. The assembly of any one of features 21-27 wherein the medical device is a urinary catheter.

## Claims

1. A urinary catheter assembly, comprising:
a package containing:
a urinary catheter having a hydrophilic coating thereon;
a protective coating over the hydrophilic coating, wherein the protective coating temporarily prevents hydration or temporarily limits the level of hydration of the hydrophilic surface, the protective coating being a liquid that has a viscosity between 30cP to 60cP at 25°C; and
an amount of hydration fluid.

2. The assembly of claim 1, wherein the hydration fluid comprises liquid water in direct liquid contact with the urinary catheter.

3. The assembly of claim 1, wherein the hydration fluid is liquid water that produces a water vapor that hydrates the hydrophilic coating.

4. The assembly of claim 3, wherein the liquid water is separated from the medical device.

5. The assembly of any one of claims 1-4, wherein the protective coating comprises a polyol.

6. The assembly of any one of claim 5, wherein the polyol comprises one or more of glycerol, propylene glycol and tetraethylene glycol.

7. The assembly of any one claims, wherein 1-6 the hydrophilic coating comprises polyvinylpyrrolidone.

8. The assembly of any one of claims 1-7, further including a sleeve within the package and wherein the catheter is positioned within the sleeve.

9. The assembly of claim 8, wherein the sleeve contains the hydration fluid.

10. A method of sterilizing a urinary catheter having a hydrophilic coating thereon, comprising:
applying a protective coating over a hydrophilic coating of a urinary catheter, wherein the protective coating temporarily prevents hydration or limits the level of hydration of the hydrophilic coating, the protective coating being a liquid that has a viscosity between 30cP to 60cP at 25°C;
placing the urinary catheter in a hydration environment; and
exposing the medical device to sterilizing radiation, prior to the hydrophilic coating being hydrated into a ready-to-use condition.

11. The method of claim 10 wherein the hydrophilic coating comprises a crosslinked hydrophilic polymer.

12. The method of any one of claims 10-11, wherein the hydrophilic coating comprises polyvinylpyrrolidone.

13. The method of any one of the claims 10-12, wherein the sterilizing radiation comprises E-beam or gamma radiation.

14. The method of any one of claims 10-13 wherein the placing the urinary catheter within a hydration environment comprises placing the urinary catheter and a hydration fluid in a package.

15. The method of claim 14 wherein the package is sealed prior to exposing the medical device to sterilizing radiation.
